# EUROPEAN PATENT SPECIFICATION

(11) **EP 2 015 727 B1**
(45) Date of publication and mention of the grant of the patent: **06.01.2010**
(21) Application number: 07776975.0
(22) Date of filing: 10.05.2007
(51) Int. Cl.: A61K 8/85, A61Q 17/04

(54) **WATER RESISTANT SUNSCREEN FORMULATIONS WITH SULFOPOLYESTERS AND PHOSPHATE ESTER SURFACTANTS**
WASSERFESTE SONNENSCHUTZFORMULIERUNGEN MIT SULFOPOLYESTERN UND PHOSPHORSÄUREESTERTENSIDEN
COMPOSITIONS D'ÉCRAN SOLAIRE RÉSISTANT À L'EAU, À BASE DE SULFOPOLYESTERS ET D'AGENTS TENSIOACTIFS D'ESTER DE PHOSPHATE

(30) Priority: 11.05.2006 US 799572 P; 08.05.2007 US 800859
(43) Date of publication of application: 21.01.2009
(73) Proprietor: EASTMAN CHEMICAL COMPANY, Kingsport TN 37660 (US)
(72) Inventor: MCENTIRE, Edward, Enns, Kingsport, TN 37664 (US); MCCAULLEY, James, Allen, Ringoes, NJ 8551 (US)
(74) Representative: Bradley, Adrian
(86) International application number: PCT/US2007/011354
(87) International publication number: WO 2007/133662

(56) References cited:
- EP-A- 1 371 355
- US-A- 5 266 322
- US-A1- 2002 197 289
- US-A1- 2003 161 792
- US-A1- 2004 151 673
- US-A1- 2004 258 644

## Description

### FIELD OF THE INVENTION

The present invention relates to sunscreen formulas having improved water resistance.

### BACKGROUND OF THE INVENTION

Sunscreen formulas, when dry on the skin, have limited water resistance when subjected to direct water contact. Wash-off due to swimming or perspiration occurs to varying degrees depending on the formulation and the nature of the UV absorbers present. In addition, organic UV absorbers are known to penetrate the stratum corneum and enter the epidermis and may even enter the dermis.

Numerous polymers have been used as materials which improve sunscreen formulas. Polymers can both increase the SPF of a sunscreen formula by assisting in the even spreading and distribution of the sunscreen filters (ultraviolet light absorbers) on the skin and increase its water resistance by both increasing the hydrophobic nature of and increasing the viscosity of the oil phase. These generally oil phase soluble polymers exist in many forms, and are known in the art as waxes, polyolefins, copolymers of olefins and vinyl pyrrolidinone, olefins and maleate esters, acrylates copolymer, silicones and others. These polymers may be effective in oil formulas, oil-in-water emulsion formulas, and water-in-oil emulsion formulas.
US 2004/151673 discloses anti-sun formulations which comprise a sulfopolyester and a UV light absorbing compound. The compositions can additionally comprise surfactants, however phosphate ester surfactants are not mentioned.
US2003/161792 discloses cosmetic sunscreen products comprising UV light absorbing compounds and phosphate ester surfactants. The compositions may also comprise polymers but sulfopolyesters are not mentioned.
EP1371355 discloses cosmetic film-forming compositions which comprise a sulfopolyester. The compositions can additionally comprise phosphate ester surfactants and UV light absorbing compounds.
Sunscreen formulations are often emulsions. Benefits are derived from both phases, each of which offer benefit to the skin and each phase may carry different ingredients to the skin. To form emulsions, surfactants are generally used to provide formulations stable toward separation. Suitable surfactants are of many types and may be non-ionic, anionic, amphoteric, or cationic. Most typical for oil in water emulsions are anionic surfactants. These surfactants are used in amounts, based on the total formulation, of from 1% to 10%. In general, the more surfactant, the more stable the formulation, and conversely, the less surfactant, the poorer the formulation stability. Also, in general, the more surfactant present, the less water resistant is the sunscreen formula after application to the skin.

The present inventors have found that the combination of a phosphate surfactant and a water dispersible sulfopolyester polymer addresses the problems discussed above. For example, the sunscreen formulas of the present invention are stable oil-in-water emulsions and require less phosphate surfactant. In addition, greater water resistance is observed over either phosphate surfactant alone, or sulfopolyester alone. The water-dispersible sulfopolyester containing formulas of the present invention are significantly better in water resistance than typical oil phase soluble polymers that have been recommended for use in sunscreen formulas.

### SUMMARY OF THE INVENTION

An embodiment of the invention concerns a composition for the absorption of solar radiation. The composition includes a sulfopolyester polymer, a phosphate ester surfactant, and an ultraviolet light absorbing compound.

Another embodiment concerns a method of making a composition for the absorption of solar radiation. The method includes mixing and heating a phosphate ester surfactant or a phosphate ester surfactant blend and an ultraviolet light absorbing compound and an oil to form an oil phase; mixing a sulfopolyester polymer and water to form an aqueous phase; combining and mixing the oil phase and the aqueous phase to form an emulsion.

Still another embodiment concerns a method of making a composition for the absorption of solar radiation. The method includes mixing a phosphate ester surfactant or a phosphate ester surfactant blend, a sulfopolyester polymer, and an ultraviolet light absorbing compound.

Yet another embodiment concerns the composition of the invention for applying to skin.

### DETAILED DESCRIPTION OF THE INVENTION

The present invention relates to sunscreen formulas having improved water resistance. The combination of a water dispersible sulfopolyester polymer and an amount of a phosphate ester surfactant or phosphate ester surfactant blend has beneficial effects on sunscreen formulas. Superior formula water resistance was found for *in vitro* testing with combination of both ingredients, indicating a synergistic influence. In fact, one oil soluble polymer was shown to detract from water resistance protection, whereas the water dispersible sulfopolyesters actually improve the water resistance. In addition, in the present invention, the sunscreen compositions include significantly less amounts of the phosphate ester surfactants or blends than those recommended by the manufacturers for stabilizing emulsions, Amounts of phosphate ester or phosphate ester blend less than 10% based on the oil phase are useful and acceptable.

Suitable sulfopolyester polymers for use in this invention are those known as Eastman AQ® polymers and Eastman AQ Copolyesters. An example of a suitable sulfopolyester is Eastman AQ38® or a sulfopolyester polymer having a glass transition temperature (Tg) or softening point of about 38°C. Eastman AQ55® or a sulfopolyester polymer having a glass transition temperature (Tg) or softening point of about 55°C would also be useful in the present sunscreen compositions. In general, suitable polymers are such polymers prepared from dimethyl-5-sodiosulfoisophthalate and its parent acid and salts, which may be derived from such co-monomers as isophthalic acid, terephthalic acid, and their esters. Diols commonly used with such acid co-monomers are diethylene glycol, ethylene glycol, triethylene glycol, polyethylene glycol, propylene glycol, 2-methyl propane diol, neopentyl glycol, 1,6-hexanediol and cyclohexane dimethanol.

The polymer can be selected from water-dispersible or water dissipating sulfopolyesters or polyesteramides (herein after referred to collectively as sulfopolyesters) containing ether groups and sulfonate groups having a glycol residue and a dicarboxylic acid residue and at least one difunctional comonomer containing a sulfonate group attached to an aromatic nucleus and in the form of a metallic salt. Such polymers are well known to those skilled in the art and are available from Eastman Chemical Company under the tradename of Eastman AQ polymers. Such sulfopolyesters can be dissolved, dispersed or otherwise dissipated in aqueous dispersions, for example at temperatures of less than about 80°C. Such polyesters are described in greater detail in U.S. Pat. No. 3,734,874 issued to Charles Kibler on May 22, 1973. One skilled in the art will understand that the term "residue" or "component- as used hereon, refers to the moiety that is the resulting product of the chemical species in a particular reaction scheme or subsequent formulation or chemical product, regardless of whether the moiety is actually obtained from the chemical species. Thus, for example, an ethylene glycol residue in a polyester refers to one or more -OCH2CH2O- repeat units in the polyester, regardless of whether ethylene glycol is used to prepare the polyester.

The aforedescribed polyester material is prepared according to the polyester preparation technology described in U.S. Pat. Nos. 3,734,874; 3,779,993; 3,828,010, 4,233,196, 5,006,598, 5,543,488, 5,552,511, 5,552,495, 5,571,876, 5,605,764, 5,709,940, 6,007,749 and 6,162,890 and the use herein of the term "acid" includes the various ester forming or condensable derivatives of the acid reactants such as the dimethyl esters thereof as employed in the preparations set out in these patents. Among the useful sulfomonomers are those wherein the sulfonate group is attached to an aromatic nucleus such as benzene, naphthalene, or biphenyl, or wherein the nucleus is cycloaliphatic such as in 9 ,4-cyclohexanedicarboxylic acid.

Suitable phosphate ester surface active materials, further referred to as phosphate ester surfactants, or blends of such surfactants with other such fatty alcohol components are of the following types: linear alcohol phosphate esters, branched alkyl alcohol phosphate esters, aralkyl phosphate esters, alkarylalkyl phosphate esters, and alkoxylated phosphate esters such as ethoxylated alcohol phosphate esters, propoxylated alcohol phosphate esters, wherein phosphate esters refers to mono-phosphate esters, di-phosphate esters, and their blends. Alkyl groups referred to above in "alcohol phosphate esters" and "alkoxylated phosphate esters" are aliphatic surfactant range alcohols, derived from 8 to 30 carbons. Likewise, the aralkyl, alkarylalkyl phosphate esters referred to above contain in said moieties groups containing from 8 to 30 carbons, again derived from the surfactant range alcohols or olefins.

Of these specialty phosphate ester surfactants, one such combination known as Grodafos® CES (available from Croda, Inc, Edison, NJ 08837), has proved especially useful. This composition is actually a surfactant blend of a phosphate surfactant, a fatty alcohol, and an ethoxylated alcohol phosphate ester. It has the following INCI name: Cetearyl Alcohol (and) Dicetyl Phosphate (and) Ceteth-10 Phosphate

Other such suitable phosphate ester combinations may be found in US Patent 6,117,915. Such materials, including those containing both phosphate esters and fatty alcohols, are thought to form liquid crystal structures in aqueous media. This feature of these materials may be responsible for the relative water resistance found. Reference to this described liquid crystal structure may be found in "Sunscreen Formulas with Multilayer Lamella Structure," T. Gao, J. Tien, and Y. Choi, Cosmetics & Toiletries Magazine, Volume 118, No. 10, October, 2003.

Other suitable phosphate ester surfactants are offered by Croda as Crodafos® CP-50 (Cetyl Phosphate (and) Stearic Acid), CS20 (Cetearyl Alcohol (and) Ceteth-20 Phosphate (and) Dicetyl Phosphate), N10 (Oleth-10 Phosphate), N5 (oleth-5 Phosphate (and) Dioleyl Phosphate), and N3 (Oleth-3 Phosphate). These may be used alone or with suitable fatty alcohols. Still other such suitable phosphate ester surfactants are offered by DSM, and known by the Amphisol trade name. An example is potassium cetyl phosphate. Another suitable phosphate ester is PPG-5 Ceteth-10 Phosphate.

Also suitable phosphate esters are phospholipids, which are a special type of phosphate surfactant, formed from four components: fatty acids, a negatively-charged phosphate group, an alcohol and a backbone. They may be phosphoglycerides, sphingomyelin, and other such related phosphate esters. Specific examples are lecithin and cephalin. Phospholipids have in common long chain aliphatic groups (fatty acid derived) of from 12-20 carbons atoms in each chain, and have two such chains attached to phosphorous through an oxygen atom on a glycerol moiety and may be amphoteric or anionic.

The combination of phosphate surfactants and sulfopolyesters is suitable with known classes of UV absorbers. A non-exhaustive list of such UV absorber classes includes Anthranilate, Benzophenone, Benzotriazole, Champhor, Cinnamate, Dibenzopy methane, Imidazole, Inorganic Particulate, Manonate, Para-aminobenzoic acid, Phenol, Phenyl triazone, Salicylate, and Triazone.

A non-exhaustive list of UV absorbers which are compatible with the combination of phosphate surfactants and sulfopolyesters includes Menthyl anthranilate, Benzophenone, Benzophenone-3, Benzophenone-4, Benzophenone-5, Benzophenone-9, Drometrizole, Drometrizole Trisiloxane, Benzylidene Champhor, Champhor Benzalkonium Methosulfate, Benzylidene Champhor Sulfonic Acid, Cinoxate, Ethylhexyl Methoxycinnamate, Ferulic Acid, Octocrylene, Butyl Methoxydibenzoylmethane, Disodium Phenyl Dibenzylimidaxole Tetrasulfonate, Phenylbenzimidazole Sulfonic Acid, Polysilicone-15, PABA, Ethylhexyl Dimethyl PABA, Digalloyl trioleate, Bis-Ethylhexyloxyphenol Methoxyphenyl Triazine, Ethylhexyl Salicylate, Homosalate, TEA Salicylate, Ethylhexyl traiazone, Methylene Bis-Benzotriazolyl Tetramethylbutylphenol, Terephthalylidene Dicamphor Sulfonic Acid, Bis-(Ethylhexyloxyphenol Methoxy)Phenyl Triazine, Titanium Dioxide, and Zinc Oxide.

The combination of phosphate surfactants and sulfopolyesters is suitable for many types of sunscreen formulas. For example, the combination is suitable for oil-in-water emulsions, for sunscreen sprays, creams, lotions, make-up and other skin and lip care products. The sulfopolyester do not contribute substantially to viscosity at the concentrations used in sunscreen oil-in-water formulas, typically 1-3%. Even much higher sulfopolyester concentrations (up to 25% of the total formula) do not contribute substantially to viscosity. Whereas the phosphate surfactants may contribute to viscosity at higher concentrations, at concentrations less than 10% of the formulation, there is some contribution to viscosity, at concentrations of 5%, less contribution to viscosity, and at 3% concentration, even less contribution to viscosity. Suitable phosphate ester surfactants are typically anionic or amphoteric surfactants. An example of a suitable phosphate ester surfactant blend includes cetearyl alcohol, dicetyl phosphate, and ceteth-10 phosphate. An example of a phosphate surfactant is cetyl phosphate, which may be in the form of a salt

When the composition of the present invention is in the form of an emulsion, the emulsion can have an oil phase in an amount from 15 to 50% or from 25 to 50%: Moreover, the phosphate ester can be less than 10% of the weight of the oil phase.

Plasticizers may be included to influence the film formation properties of the sulfopolyester and which may influence delivery rate of ultraviolet absorbers to the skin. These plasticizers include but are not limited to ingredients such as propylene glycol, glycerin, methyl pentane diol, butylene glycol, hexylene glycol, octanediol, phenoxyethanol, cyclohexane dimethanol, glycolic acid, lactic acid, acetic acid, propionic acid, salicylic acid, triethyl citrate, acetoxy triethyl citrate, tributyl citrate, triacetin, glycerol esters of acetic, propionic acid, and mixtures thereof.

The typical level of plasticizer incorporated in the formulation, based on the weight of the sulfopolyester polymer, is from zero to 25% or more, Indeed, the level of plasticizers may have to be limited so that less UV absorber is delivered to the skin surface. Usually, the plasticizer is used in an effective amount to keep the polymer film from cracking, flaking, or otherwise being prematurely removed from the skin, but not so much that the film formed becomes tacky to the touch. Another way to gauge how much plasticizer is needed is to add various levels of plasticizer to the polymer dispersion, then measure the Tg of the film formed therefrom. If the polymer film has a Tg (as measured by DSC of the dry film [dried for at least 8 hours at ambient temperature] from the polymer dispersion) of 40 degrees C or lower, little or no plasticizer is required. However, if the Tg of the dry polymer film is greater than 40 degrees C, to achieve good film formation and films with sufficient elongation, plasticizer may be added to reduce the Tg to 40 degrees or less, to 30 degrees C or less, and even to 25 degrees C or less, which is determined experimentally. Once the plasticizer has been added to the formula so that a dry film Tg of 40 degrees C or less is obtained, then the film will have a minimum tendency to crack or flake from the skin.

To gage the maximum amount of plasticizer to add to a formulation, if the plasticizer amount causes the film to be tacky or to have a very high friction surface, then too much plasticizer has been added. A guideline is that the Tg of the polymer film should be above -20 degrees C, or above -10, and even at or above zero degrees C.

However, added plasticizer may be un-necessary, since the UV absorber may act also as a plasticizer for the polymer, reducing the Tg of the film formed on the skin (or other substrate) to 40 degrees or less. This may also be determined experimentally, and depends on the desired UV absorbers used in the formula.

Typical use levels of sulfopolyester and phosphate surfactant based on the total formula weight, are from 1 to 25%, from 1 to 10% or from 1 to 5% sulfopolyester, and for the phosphate surfactant, from 1% to 5%, more preferably from 1% to 4% and even more preferably from 1 to 3%.

The advantages of the combination of the phosphate surfactant and sulfopolyester over the prior art are that the formulas are stable oil-in-water emulsions. The emulsions are generally stable for longer than 60 days. The composition of the present invention also require less phosphate surfactant than amounts required by the manufacture of the surfactant for stabilizing emulsions. Moreover, greater water resistance (including sea water) is observed over either phosphate surfactant alone, or sulfopolyester alone. The sulfopolyester containing formulas are signifcantly better in water resistance than typical oil phase soluble polymers that have been recommended for use in sunscreen formulas.

Sulfopolyesters with phosphate ester surfactants are also very water resistant in sea water, or water of high ionic strength. Sea water typically contains inorganic salts such as sodium chloride, potassium chloride, and magnesium salts in total concentration of 3.5% by weight. Thus ocean swimming will have less of an effect on the UV absorbers held within the polymer/surfactant matrix on the skin after application from a sunscreen formula than with other polymer-surfactant combinations.

In addition, UV absorbers are less likely to be removed by perspiration when present on the skin with sulfopolyesters in combination with phosphate ester surfactants. Perspiration often has the unfortunate circumstance of washing into the eyes UV absorbers that have been applied to the face from a formula. Since perspiration has a high ionic strength the sulfopolyester and phosphate ester surfactant combination help retain the UV absorbers on the skin where they were applied from the sunscreen or face treatment formula. Typical ionic strength of perspiration is from 50 to 130 millimoles/liter, and consisting of sodium chloride, sodium lactate, potassium chloride, and other organic salts. Additional compounds are typically contained such as bicarbonates and urea.

In addition, sulfopolyesters with phosphate ester surfactants in sunscreen formulas retain more of the UV absorber at the skin surface than a similar formula applied from formulas without sulfopolyesters, The sulfopolyesters appear to incorporate organic UV absorbers within the polymer film on the skin, where the UV absorbers prefer to reside, rather than penetrate into the skin.

Other ingredients may be included in sunscreen formulations, such as emollients, fragrance, preservatives, chelating agents, neutralants to adjust pH, buffer, organic or inorganic ultraviolet light absorbers, dyes or pigments, alcohols such as ethanol, silicones, skin lubricants, skin nutrients, and vitamins.

In general, the sunscreen compositions of the present invention are produced by mixing and heating at up to 80 degrees C oil phase ingredients, generally including UV absorber, phosphate surfactant, and other oil soluble ingredients such as emollients. When the oil phase ingredients are in solution, the oil is poured with mixing into the heated water phase containing the sulfopolyester and other water soluble ingredients such as preservatives, chelating agents. The mixing may be low shear stirring or high shear homogenization such as that provided by high speed rotor-stator mixers and impingement mixers. The aqueous phase may also be heated at up to 80 degrees C, and the mixture may also be heated at up to 80 degrees C.

Other suitable procedures include pouring the aqueous phase into the heated oil phase, again, optionally with heating. Heating may be also provided by the high shear mixing process which also provides heat by friction. The mixing may be high or low shear depending on factors known by those skilled in the art, and is generally influenced by the nature of the surfactant, the solubility of the UV absorber, additives, ionic content and stirrer configuration and type. Once the two phases are suitably mixed, other ingredients may be added, such as preservatives, neutralizing agentes and buffers.

### EXAMPLES:

### Examples 1- 5 - Formulas

The following formulas were prepared by blending and heating the oil soluble ingredients, then under mixing conditions provided by a high shear rotor-stator mixer (Janke and Kunkel GMBH, IKA-Laborkechnik Ultra-Turrax T50) set at maximum speed, the oil phase was poured slowly into the aqueous phase containing the remaining ingredients of glycerin, EDTA, AQ38 polymer (predispersed in water) and water, and high shear continued for 10 minutes. The blend was then placed on slow stirring by a paddle blade stirrer until cooled to at least 35 degrees C. Triethanolamine was added to adjust to a final pH of 6.

| **Experiment Number** | **1** | **2** | **3** | **4** | **5** |
|---|---|---|---|---|---|
| **Ingredients** | | | | | |
| C12-15 Alkyl Benzoate | 5% | 5% | 5% | 5% | 5% |
| (Finsolv® TN, Fintex) | | | | | |
| Octyl Methoxy | 7% | 7% | 7% | 7% | 7% |
| Cinnamate (Escalol® 557, ISP) | | | | | |
| Butyloctyl Salicylate | 2% | 2% | 2% | 2% | 2% |
| (Hallbrite® BHB, RTD Hall Star, Inc.) | | | | | |
| Octocrylene (Neo Heilopan® 303, Symrise) | 2% | 2% | 2% | 2% | 2% |
| Benzophenone-3 | 4% | 4% | 4% | 4% | 4% |
| (Escalol 567, ISP) | | | | | |
| Avobenzone (Parsol® 1789, DSM) | 2% | 2% | 2% | 2% | 2% |
| Cetearyl alcohol and Dicetyl phosphate and Ceteth-10 Phosphate | 2% | 2% | 2% | 2% | 2% |
| (Crodafos® CES, Croda, Inc.) | | | | | |
| Myristyl Myristate | 3% | 3% | 3% | 3% | 3% |
| (Crodamol® MM, Croda, Inc.) | | | | | |
| Glyceryl Stearate and Glycerin (Cerasynt SD, ISP) | 1% | 1% | 1% | 1% | 1% |
| C30-38 | 0% | 1% | 0% | 1% | 0% |
| Olefin/lsopropyl | | | | | |
| Maleate/MA copolymer | | | | | |
| (Pertorma® V-1608) | | | | | |
| PVP/Eicosene Copolymer (Antaron V-220F, ISP) | 0% | 0% | 0% | 0% | 1% |
| Glycerin | 3% | 3% | 3% | 3% | 3% |
| EDTA.2Na.2H2O | 0.05% | 0.05% | 0.05% | 0.05% | 0.05% |
| Triethanolamine | q.s. | q.s. | q.s. | q.s. | q.s. |
| Eastman AQ38® | 0% | 0% | 3% | 3% | 0% |
| Polymer | | | | | |
| Water | 69% | 69% | 66% | 65% | 65% |
| Total | 100.05% | 100.05% | 100.05% | 100.05% | 100.05% |

| | | | | | |
|---|---|---|---|---|---|
| q.s. = quantity sufficient for formulation pH = 6+/-0.2 | | | | | |

The emulsions were stable when tested at 54 degrees C for two months. All samples were low enough in viscosity to be atomized into a fine spray with a pump spray nozzle from a container.

### In vitro Water Resistance Testing of Examples 1-5 (Tests conducted and described by a recognized sunscreen test and development laboratory).

Samples of each sunscreen spray formula were tested for SPF and water resistance by carefully spreading with a gloved fingertip at the rate of 1.5 mg/cm² the sunscreen formula onto a roughened poly(methyl methacrylate) slide (available from Heiloscience.com). Samples were allowed to dry for 15 minutes, then measured for SPF using a Labsphere UV-1000S Ultraviolet Transmittance Analyzer. Samples were then immersed in a container filled with tap water while the water was stirred gently, and remained therein for 20 minutes. The panels were recovered from the bath, allowed to dry, then re-immersed in the stirring water for 20 minutes again, removed and allowed to dry. The SPF was re-measured and compared to that originally measured for the sample.

The percent retention of SPF after water immersion for each sample was recorded, and is shown in the table below. By comparison with commercial control sunscreens, the samples were judged to have an SPF of about 30.

| **Formula of Experiment No.** | **Percent SPF Retention** |
|---|---|
| 1 | 84 |
| 2 | 84 |
| 3 | 96 |
| 4 | 88 |
| 5 | 87 |

As may be seen from the data, the best water resistance (or SPF retention) is observed for the AQ38 containing formula.

### Examples 6- 9

Formulas prepared exactly as those in Examples 1-5 were made, but the polymers in the formulas above were eliminated and replaced with those following so that the only differences in the formulas are as noted in the table below:

| **Example No.** | **6** | **7** | **8** | **9** |
|---|---|---|---|---|
| Ingredient | | | | |
| PVP/Eicosene Copolymer (Antaron V-220F, ISP) | 1% | 0% | 2% | 0% |
| Eastman AQ38 | 0% | 1% | 0% | 2% |

Water resistance was determined as for the preceding five examples, and is shown in the table below. For comparison, a commercial sunscreen purchased in 2005 was subjected to the same test conditions as a control. This SPF 30 product from a major consumer products company had a percent SPF retention of 46%.

| **Formula of Experiment No.** | **Percent SPF Retention** |
|---|---|
| 6 | 86.6 |
| 7 | 92.4 |
| 8 | 89.5 |
| 9 | 96.6 |

Again the best water resistance and SPF retention was observed as a result of the AQ38 polymer.

### Example 10

A formula identical to that of Example 3 was prepared, except 2% cetyl phosphate, potassium salt, Amphisol® K (available from DSM) was substituted for 2% of Crodafos CES. The sample proved stable to storage for over six months at ambient temperature and stable to accelerated storage in an oven at 54C for at least 2 months.

### Example 11

A formula identical to that of Example 3 is prepared, except that 1 % of triethyl citrate is added to plasticize the polymer, and Eastman AQ55 is substituted for AQ38 in the same amount.

### Example 12

The formula from Example 1 is tested for mobility of the UV absorbers, by applying to the facial skin on one side of the face at the rate of approximately 2 mg/cm² to a 4 cm² patch of skin adjacent to the eye, at approximately 1 cm from the side of the eye. This application is compared with a similar application of the formula of Example 3 to facial skin on the opposite side of the face. Under hot and humid conditions for 30 minutes, perspiration of the facial skin occurs. Upon removal from the hot and humid conditions, perspiration ceases. Examination of the areas to which the sunscreen formulas from Examples 1 and 3 are applied by using a UV lamp illuminating with 365 nm ultraviolet light reveals that the area to which Example 1 is expanded to a larger area of approximately 5 cm² because of lateral migration of the UV absorbers on the skin. However, the UV absorbers from formula 3 are observed to be in the same area to which they were applied, so little or no migration of the UV absorbers occurs in this product containing AQ38 polymer.

Comparison of the formula of Examples 8 and 9 in this same comparison on the skin show little or no migration of the UV absorbers with the AQ38 containing formula of Example 9, but show some migration of the UV absorbers of Example 8 containing the control polymer, Antaron V220.

### Example 13

Preparation of Sulfopolyester A. A round bottom flask equipped with ground-glass head, an agitator shaft, nitrogen inlet and a side arm was charged with 82 mole percent isophthalic acid, 18 mole percent dimethyl-5-sodiosulfoisophthalate (SIP), 54 mole percent diethylene glycol (DEG), and 46 mole percent 1,4-cyclohexanedimethanol (CHDM), based on 100 mole percent dicarboxylic acid and 100 mole percent diol. A catalyst was added and the flask was immersed in a Belmont bath at 200°C for one hour under a nitrogen sweep. The temperature of the bath was increased to 230°C for one hour. After one hour the temperature of the bath was increased to 280°C and the flask was heated for 45 minutes longer under a reduced pressure of 0.5 to 0.1 mm of Hg. The flask was allowed to cool to room temperature. The copolyester was removed from the flask and ground to less than 3 mm granules. Sulfopolyester A had a Tg of 53°C.(as determined by differential scanning calorimetery) and an Inherent Viscosity (I.V.) of 0.33 dl/g was measured at 23°C using 0.50 grams of polymer per 100 ml of a solvent consisting of 60% by weight phenol and 40% by weight tetrachloroethane.

A dispersion of the Sulfopolyester A polymer granules was prepared by heating to 80°C 136 grams of deionized water in a 500 milliliter beaker. Then 64 grams of the polymer granules were added with stirring, and the stirring continued for 30 minutes. The weight of the water that evaporated on heating was replaced as the formula cooled, giving a nearly clear polymer dispersion.

### Example 14

In accordance with the present invention, a formulation that provides a film on the skin which contains an ultraviolet light absorber was prepared by combining the following constituents: (a) 47.7 grams of the dispersion of Example 13; and (b) 2.61 g of triethyl citrate. These were blended together to produce 50.3g of a polymer-plasticizer blend. To this was added (c) 4.08 grams of ethylhexyl methoxycinnamate (Uninul® MC-80, available from BASF, Inc). After additional blending and heating to 60°C for one hour, the blend was then cooled. A viscous, slightly opaque dispersion was produced.

The dispersion was applied to the skin of the volar forearm of a test subject at the rate of 0.01 grams per square centimeter using a small brush. The liquid dried rapidly (less than five minutes) to a film. The dry film remained on the skin for 3 hours, and was then removed, collected completely and analyzed by liquid chromatography for ethylhexyl methoxycinnamate. The film retained most (69-78% in two experiments) of the ultraviolet light absorbing chemical.

Conversely, when the active ingredient, ethylhexyl methoxycinnamate, was applied to the skin at nearly twice the dosage to that above, 0.00143 grams per square centimeter, the entire amount of active ingredient was absorbed into the skin within one hour. This demonstrates that much less of an ultraviolet light absorbing chemical is absorbed into the skin when the chemical is contained in the dry film, even though the formulation is applied as a liquid.

### Example 15

### A1. This example illustrates the use and formula water-resistance of substantially water-insoluble UV absorbers in combination with a sulfopolyester.

An oil phase was prepared containing the UV absorbers by adding sequentially the following ingredients with stirring and warming to about 80 degrees C.

| **Ingredients** | **Weight %** |
|---|---|
| C12-15 Alkyl Benzoate (Finsolv® TN from Finetex, Inc.) | 5 |
| Butyloctyl Salicylate (Hallbrite®BHB from RTD HallStar, Inc) | 2 |
| Ethylhexyl methoxycinnamate (Parsol® MCX from DSM) | 7.5 |
| Octacrylene (Parsol 340 from DSM) | 2.39 |
| Octyl Salicylate (Eusolex® OS from Merck) | 5 |
| Avobenzone (Parsol 1789 from DSM) | 3 |
| Myristyl Myristate (Crodamol® MM, Croda) | 3 |
| Glyceryl Stearate (Cutina® GMS V, Cognis) | 3 |
| Cetearyl Alcohol (Lanette® O Cognis) | 1 |

Then an aqueous phase was prepared by first combining the water and all ingredients except the AQ polymer and warming to about 75 degrees C.

| | |
|---|---|
| Demineralized Water | 60.02 |
| Eastman AQ®38S Water Dispersible Sulfopolyester | 2 |
| Glycerin | 3 |
| Disodium EDTA (Versene®NA2 chelating agent from Dow) | 0.05 |
| Potassium Cetyl Phosphate (Amphisol K®, DSM) | 2 |
| Aqua, Sodium Hydroxide (NaOH 30% aq solution) | 0.04 |

The AQ38 polymer pellets were then added and stirring continued until dissolved. The oil phase was then added to the aqueous phase while stirring at high speed to form a pre-emulsion. Then the warm blend was then homogenized for 10 minutes using a rotor-stator homogenizer, and then slow stirring was resumed until the emulsion was cooled to ambient temperature. The preservative was then stirred into the emulsion.
Preservative Blend (Phenonip® from Clariant) 1

The pH was checked and adjusted if necessary with sodium hydroxide to between 6 and 7.

### A2. A sunscreen lotion control was prepared which was identical to that above, but wherein the Eastman AQ38 polymer was replaced by water.

### Example 16

### B. This example illustrates the use and formula water-resistance of a water-soluble UV absorber in combination with a sulfopolyester.

An oil phase was prepared containing the oil soluble UV absorbers by adding sequentially the following ingredients with stirring and warming to about 75 degrees C.

| **Ingredient (INCl Name)** | **Weight Percent** |
|---|---|
| C12-15 Alkyl Benzoate | 4.89 |
| Ethylhexyl Methoxycinnamate | 7.34 |
| Octocrylene | 2.74 |
| Avobenzone | 2.94 |
| Diethylhexyl naphthoate (Corapan®, TQ, Symrise GmbH & Co.) | 2.94 |
| Myristyl Myristate | 2.94 |
| Glyceryl Stearate | 2.94 |
| Cetearyl Alcohol | 0.98 |

An aqueous phase was also prepared by combining the ingredients below, including the water-soluble UV absorber. First a dispersion of Eastman AQ38 polymer was prepared by first warming a portion of the water below (4.57 %) to 70 degrees C, then adding Eastman AQ38 polymer pellets (Polyester-5) and stirring until the pellets were dissolved. The remaining water and other ingredients were then added sequentially which dissolved to form a clear dispersion, using the final sodium hydroxide solution to adjust pH to 6.1.

| **Ingredient (INCl Name)** | **Weight Percent** |
|---|---|
| Aqua | 57.16 |
| Benzophenone-4 (Uvinul® MS-40 from BASF, Inc.) | 4.89 |
| Glycerin | 2.94 |
| Disodium EDTA | 0.05 |
| Potassium Cetyl Phosphate | 1.96 |
| Preservative (Phenonip® from Clariant, Inc.) | 0.98 |
| Polyester-5 (Eastman AQ®38S Water Dispersible Sulfopolyester) | 1.96 |
| Aqua, Sodium Hydroxide (30% solution) | 2.35 |

The warm (80°C) oil phase was poured into the warm (70°C) aqueous phase with rapid stirring. The combined emulsion blend was then homogenized by mixing at 3000 rpm for 10 minutes on a Janke & Kunkel IKA-Labortechnik Ultra-Turrax, T-50 Model having a mixing head diameter of 4.5 cm and a slit opening of 0.2 cm. The resultant oil-in-water emulsion had a pH of 6.2.

The sunscreen lotion formula samples were submitted for SPF testing to the independent clinical testing lab, AMA Laboratories, New City, NY. Using FDA protocols, they ran blind testing on five subjects using the submitted formulas for both Static SPF (before any subjects were exposed to water) and Very Water Resistant SPF (after the subjects received four 20 minute immersions in water with air-drying periods following each water immersion). The percent SPF retention following water exposure was calculated by dividing the Very Water Resistant SPF by the Static SPF, then multiplying the result by 100. This value is thought to simulate the SPF protection that one may expect after applying a sunscreen formula, allowing it to dry, and then being exposed to water by swimming or perspiring.

The results of the *in vivo* testing are presented for the sunscreen lotions in the following table. Included for comparison was a commercial SPF 30 sunscreen sold in 2005 from a leading US supplier advertised as 'waterproof with UVA protection.

| **Composition of Example** | **Static SPF** | **VWR* SPF** | **% SPF Retention** |
|---|---|---|---|
| **A1** | 30.8 | 29.2 | 94.8 |
| **A2** (Control) | 29.2 | 27.2 | 93.2 |
| **B** | 31.3 | 28.8 | 92.0 |
| Commercial SPF 30 Lotion | 30.8 | 28.7 | 93.2 |

| | | | |
|---|---|---|---|
| * VWR = Very Water Resistant protocol | | | |

Note that all except the control had high static SPF, and all had very good SPF retention following water exposure to the very water resistant protocol. Even the formula from Example B containing a significant portion of a water-soluble UV absorber (Benzophenone-4) provided excellent VWR SPF and °/o SPF retention.

## Claims

1. A composition for the absorption of solar radiation comprising:
a) a sulfopolyester polymer;
b) a phosphate ester surfactant or a phosphate ester surfactant blend; and
c) an ultraviolet light absorbing compound.

2. The composition according to claim 1, wherein the composition is an emulsion having an oil phase in an amount of from 15 to 50%, and wherein said surfactant is less than 10% of the weight of the oil phase.

3. The composition according to claim 2, wherein the amount of sulfopolyester is from 1 % to 5% based on a total weight of the composition.

4. The composition according to claim 1, wherein the surfactant includes at least one of cetearyl alcohol, dicetyl phosphate, ceteth-10 phosphate and cetyl phosphate.

5. The composition according to claim 1, further comprising a plasticizer selected from the group consisting of propylene glycol, glycerin, methyl pentane diol, butylene glycol, hexylene glycol, octanediol, phenoxy ethanol, cyclohexane dimethanol, glycolic acid, lactic acid, acetic acid, propionic acid, salicylic acid, diethyl tartrate, triethyl citrate, acetoxy triethyl citrate, tributyl citrate, triacetin, glycerol esters of acetic and propionic acid.

6. The composition according to claim 1, further comprising at least one fatty alcohol.

7. The composition according to claim 1, wherein the surfactant is a liquid crystal forming surfactant.

8. The composition according to claim 1, wherein the composition is in the form of an emulsion, a spray, a cream, a lotion, a make-up, and a lip-care product.

9. A method of making a composition for the absorption of solar radiation comprising:
mixing and heating a phosphate ester surfactant or a phosphate ester surfactant blend and an ultraviolet light absorbing compound and an oil to form an oil phase;
mixing a sulfopolyester polymer and water to form an aqueous phase; combining and mixing the oil phase and the aqueous phase to form an emulsion.

10. The method according to claim 9, wherein the oil phase is in an amount of from 15 to 50%, and wherein the surfactant is less than 14% of the weight of the oil phase.

11. The method according to claim 9, wherein the amount of sulfopolyester is from 1% to 5°/d based on a total weight of the composition.

12. The method according to claim 9, wherein the surfactant includes at least one of cetearyl alcohol, dicetyl phosphate, ceteth-10 phosphate and cetyl phosphate.

13. The method according to claim 9, wherein said composition includes a plasticizer selected from the group consisting of propylene glycol, glycerin, methyl pentane diol, butylene glycol, hexylene glycol, octanediol, phenoxy ethanol, cyclohexane dimethanol, glycolic acid, lactic acid, acetic acid, propionic acid, salicylic acid, diethyl tartrate, triethyl citrate, acetoxy triethyl citrate, tributyl citrate, triacetin, glycerol esters of acetic and propionic acid.

14. A method of making a composition for the absorption of solar radiation, comprising mixing a phosphate ester surfactant or a phosphate ester surfactant blend, a sulfopolyester polymer, and an ultraviolet light absorbing compound.

15. The composition according to claim 1 for application to the skin.

## Patentansprüche

1. Zusammensetzung für die Absorption von Sonnenstrahlung, umfassend:
a) ein Sulfopolyesterpolymer;
b) ein Phosphatestertensid oder ein Phosphatestertensid-Blend; und
c) eine Verbindung, die ultraviolettes Licht absorbiert.

2. Zusammensetzung nach Anspruch 1, wobei die Zusammensetzung eine Emulsion ist, die eine Ölphase in einer Menge von 15 bis 50 % aufweist und wobei das genannte Tensid weniger als 10 % des Gewichts der Ölphase ausmacht.

3. Zusammensetzung nach Anspruch 2, wobei die Menge an Sulfopolyester von 1 % bis 5 %, basierend auf dem Gesamtgewicht der Zusammensetzung, ausmacht.

4. Zusammensetzung nach Anspruch 1, wobei das Tensid aus Cetearylalkohol, Dicetylphosphat, Ceteth-10-Phosphat und Cetylphosphat mindestens eines enthält.

5. Zusammensetzung nach Anspruch 1, weiterhin umfassend einen Weichmacher, der aus der Gruppe, bestehend aus Propylenglykol, Glycerin, Methylpentandiol, Butylenglykol, Hexylenglykol, Octandiol, Phenoxyethanol, Cyclohexandimethanol, Glykolsäure, Milchsäure, Essigsäure, Propionsäure, Salicylsäure, Diethyltartrat, Triethylcitrat, Acetoxytriethylcitrat, Tributylcitrat, Triacetin, Glycerolester der Essig- und Propionsäure ausgewählt ist.

6. Zusammensetzung nach Anspruch 1, die weiterhin mindestens einen Fettalkohol umfasst.

7. Zusammensetzung nach Anspruch 1, wobei das Tensid ein flüssigkristallbildendes Tensid ist.

8. Zusammensetzung nach Anspruch 1, wobei die Zusammensetzung in Form einer Emulsion, eines Sprays, einer Creme, einer Lotion, eines Make-up oder eines Lippenpflegeprodukts vorliegt.

9. Verfahren zur Herstellung einer Zusammensetzung für die Absorption von Sonnenstrahlung, umfassend:
Mischen und Erhitzen eines Phosphatestertensids oder eines Phosphatestertensid-Blends und einer Verbindung, die ultraviolettes Licht absorbiert, und eines Öls, um eine Ölphase herzustellen;
Mischen eines Sulfopolyesterpolymers und Wasser, um eine wässrige Phase herzustellen;
Kombinieren und Mischen der Ölphase und der Wasserphase, um eine Emulsion herzustellen.

10. Verfahren nach Anspruch 9, wobei die Ölphase in einer Menge von 15 bis 50 % vorliegt und wobei das Tensid weniger als 10 % des Gewichts der Ölphase ausmacht.

11. Verfahren nach Anspruch 9, wobei die Menge an Sulfopolyester von 1 % bis 5 %, basierend auf dem Gesamtgewicht der Zusammensetzung, ausmacht.

12. Verfahren nach Anspruch 9, wobei das Tensid aus Cetearylalkohol, Dicetylphosphat, Ceteth-10-Phosphat und Cetylphosphat mindestens eines enthält.

13. Verfahren nach Anspruch 9, wobei die genannte Zusammensetzung weiterhin einen Weichmacher umfasst, der aus der Gruppe, bestehend aus Propylenglykol, Glycerin, Methylpentandiol, Butylenglykol, Hexylenglykol, Octandiol, Phenoxyethanol, Cyclohexandimethanol, Glykolsäure, Milchsäure, Essigsäure, Propionsäure, Salicylsäure, Diethyltartrat, Triethylcitrat, Acetoxytriethylcitrat, Tributylcitrat, Triacetin, Glycerolester der Essig- und Propionsäure ausgewählt ist.

14. Verfahren zur Herstellung einer Zusammensetzung für die Absorption von Sonnenstrahlung, umfassend Mischen eines Phosphatestertensids oder eines Phosphatestertensid-Blends, eines Sulfopolyesterpolymers und einer Verbindung, die ultraviolettes Licht absorbiert.

15. Zusammensetzung nach Anspruch 1 zur Anwendung auf der Haut.

## Revendications

1. Composition servant à absorber les rayonnements solaires, comprenant :
a) un polymère de sulfopolyester ;
b) un tensioactif de type ester de phosphate ou un mélange de tensioactifs de type ester de phosphate ; et
c) un composé absorbant la lumière ultraviolette.

2. Composition selon la revendication 1, dans laquelle la composition est une émulsion possédant une phase huileuse en une quantité de 15% à 50%, et dans laquelle ledit tensioactif représente moins de 10% du poids de la phase huileuse.

3. Composition selon la revendication 2, dans laquelle la quantité de sulfopolyester est de 1% à 5% sur la base du poids total de la composition.

4. Composition selon la revendication 1, dans laquelle le tensioactif comprend au moins un composé choisi parmi l'alcool cétéarylique, le phosphate de dicétyle, le céteth-10 phosphate et le phosphate de cétyle.

5. Composition selon la revendication 1, comprenant en outre un plastifiant choisi dans le groupe constitué par le propylène glycol, la glycérine, le méthyl pentane diol, le butylène glycol, l'hexylène glycol, l'octanediol, le phénoxy éthanol, le cyclohexane diméthanol, l'acide glycolique, l'acide lactique, l'acide acétique, l'acide propionique, l'acide salicylique, le tartrate de diéthyle, le citrate de triéthyle, l'acétoxycitrate de triéthyle, le citrate de tributyle, la triacétine, les esters de glycérol de l'acide acétique et propionique.

6. Composition selon la revendication 1, comprenant en outre au moins un alcool gras.

7. Composition selon la revendication 1, dans laquelle le tensioactif est un tensioactif formant un cristal liquide.

8. Composition selon la revendication 1, dans laquelle la composition est sous la forme d'une émulsion, d'une pulvérisation, d'une crème, d'une lotion, d'un maquillage et d'un produit de soin des lèvres.

9. Procédé de fabrication d'une composition servant à absorber les rayonnements solaires, comprenant :
le mélange et le chauffage d'un tensioactif de type ester de phosphate ou d'un mélange de tensioactifs de type ester de phosphate, d'un composé absorbant la lumière ultraviolette et d'une huile pour former une phase huileuse ;
le mélange d'un polymère de sulfopolyester et d'eau pour former une phase aqueuse ;
la combinaison et le mélange de la phase huileuse et de la phase aqueuse pour former une émulsion.

10. Procédé selon la revendication 9, dans lequel la phase huileuse est en une quantité de 15% à 50%, et dans lequel ledit tensioactif représente moins de 10% du poids de la phase huileuse.

11. Procédé selon la revendication 9, dans lequel la quantité de sulfopolyester est de 1% à 5% sur la base du poids total de la composition.

12. Procédé selon la revendication 9, dans lequel le tensioactif comprend au moins un composé choisi parmi l'alcool cétéarylique, le phosphate de dicétyle, le céteth-10 phosphate et le phosphate de cétyle.

13. Procédé selon la revendication 9, dans lequel ladite composition comprend un plastifiant choisi dans le groupe constitué par le propylène glycol, la glycérine, le méthyl pentane diol, le butylène glycol, l'hexylène glycol, l'octanediol, le phénoxy éthanol, le cyclohexane diméthanol, l'acide glycolique, l'acide lactique, l'acide acétique, l'acide propionique, l'acide salicylique, le tartrate de diéthyle, le citrate de triéthyle, l'acétoxycitrate de triéthyle, le citrate de tributyle, la triacétine, les esters de glycérol de l'acide acétique et propionique.

14. Procédé de fabrication d'une composition servant à absorber les rayonnements solaires, comprenant le mélange d'un tensioactif de type ester de phosphate ou d'un mélange de tensioactifs de type ester de phosphate, d'un polymère de sulfopolyester et d'un composé absorbant la lumière ultraviolette.

15. Composition selon la revendication 1, pour une application sur la peau.
